# EUROPEAN PATENT SPECIFICATION

(11) **EP 1 346 045 B1**
(45) Date of publication and mention of the grant of the patent: **19.10.2005**
(21) Application number: 01998634.8
(22) Date of filing: 28.11.2001
(51) Int. Cl.: C12N 15/12

(54) **HUMAN PROTOONCOGENE AND PROTEIN ENCODED THEREIN**
MENSCHLICHES PROTOONKOGEN UND DARIN CODIERTES PROTEIN
PROTOONCOGENE HUMAIN ET PROTEINE CODEE DANS CELUI-CI

(30) Priority: 28.11.2000 KR 2000071208
(43) Date of publication of application: 24.09.2003
(73) Proprietor: Kim, Jin Woo, Seoul 135-110 (KR)
(72) Inventor: Kim, Jin Woo, Seoul 135-110 (KR)
(74) Representative: Wibbelmann, Jobst
(86) International application number: PCT/KR2001/002053
(87) International publication number: WO 2002/044371

(56) References cited:
- KR-A- 2001 039 556
- LIN LIZHU ET AL: "Cloning, tissue expression pattern, and chromosome location of a novel human gene BRI3BP" BIOCHEMICAL GENETICS, vol. 39, no. 11-12, December 2001 (2001-12), pages 369-377, XP009037989 ISSN: 0006-2928
- WU HAOQUAN ET AL: "bri3, a novel gene, participates in tumor necrosis factor-alpha-induced cell death." BIOCHEMICAL AND BIOPHYSICAL RESEARCH COMMUNICATIONS, vol. 311, no. 2, 14 November 2003 (2003-11-14), pages 518-524, XP002300188 ISSN: 0006-291X
- DATABASE EMBL HCCR-1 pro-oncogene binding protein 20 November 2001 (2001-11-20), XP002300189 retrieved from EBI Database accession no. AF283671
- DATABASE EMBL BRI3BP 20 January 2002 (2002-01-20), XP002300190 retrieved from EBI Database accession no. Q8WY22
- NGAN H.Y. ET AL: 'Proto-oncogenes and p53 protein expression in normal cervical stratified squamous epithelium and cervical intra-epithelial neoplasia' EUR. J. CANCER vol. 35, no. 10, October 1999, pages 1546 - 1550, XP002974542
- UTRERA-BARILLAS D. ET AL: 'H-ras and Nm23-H1 gene expression and proteolytic activity in squamous cell carcinoma of the uterine cervix' ARCH. MED. RES. vol. 31, no. 2, March 2000 - April 2000, pages 172 - 181, XP002974541
- MADRIGAL M. ET AL: 'In vitro antigene therapy targeting HPV-16 E6 and E7 in cervical carcinoma' GYNECOL. ONCOL. vol. 64, no. 1, January 1997, pages 18 - 25, XP002968345
- LAPPALAINEN K. ET AL: 'Effects of liposomal antisense oligonucleotides on mRNA and protein levels of the HPV 16 E7 oncogene' ANTICANCER RES. vol. 16, no. 5A, September 1996 - October 1996, pages 2485 - 2492, XP002974543

## Description

### Field of the Invention

The present invention relates to a novel protooncogene and protein encoded therein, which can be used in diagnosis of various cancers, preparation of transgenic animal, antisense gene therapy and anticancer drug development.

### Background of the Invention

Higher animals including man each carry approximately 100,000 genes, but only about 15% thereof is expressed, and characteristics of individual's biological processes, e.g., genesis, differentiation, homeostasis, responses to stimuli, control of cell division cycle, aging and apoptosis (programmed cell death), are determined depending on which genes are expressed (Liang, P. and A. B. Pardee, *Science* **257**: 967-971, 1992).

Pathogenic phenomena such as tumorigenesis are caused by gene mutation which brings about changes in the mode of gene expression. Therefore, comparative studies of gene expressions in various cells have been conducted to provide bases for establishing viable approaches to the understanding of diverse biological phenomena.

It has been reported that tumorigenesis is caused by various genetic changes such as the loss of chromosomal heterozygosity, activation of oncogenes and inactivation of tumor suppressor genes, e.g., p53 gene (Bishop, J. M., *Cell* **64**: 235-248, 1991; and Hunter, T., *Cell* **64**: 249-270, 1991). Further, it has been reported that 10 to 30% of human cancer arises from the activation of oncogene through amplification of protooncogenes.

Therefore, the activation of protooncogenes plays an important role in the etiology of many tumors and there has existed a need to identify protooncogenes.

The present inventors have endeavored to unravel the mechanism involved in the tumorigenesis of cervical cancer; and, have unexpectedly found that novel protooncogenes, human cervical cancer proto-oncogene 1 (HCCR-1) (Korea Patent Application No. 2000-16757 (March 31, 2000)) and HCCR-2 (Korea Patent Application No. 2000-71202 (November 28, 2000)), is specifically overexpressed in cancer cells. Protooncogene causes quantitative and qualitative changes of the corresponding protein product expression via a conformational change (Weinberg, R. A., *Cancer Research* 49:3713-3721, 1989). The conformationally changed-cancer protein destroys signal transduction pathways in the cell surface, cytoplasm or nucleus; while in the absence of tumor suppressor gene product, it stimulates cell proliferation together with other cancer proteins, disturbs cell cycle, and destroys cell death (apoptosis) mechanism (Todd, R., *Anticancer Research* 19:4729-4746, 2000).

The yeast two-hybrid method, developed in 1989 by Fields et al. (Fields, S. and Song, O., Nature, 340:245-246, 1989) for examining such protein-protein interactions, has been widely applied to elucidate the cell death mechanism (Wallach, D. et al., *Curr. Opin. Immunol.* 10:131-136, 1989) as well as other biological process using various genetic tools (Pandey, A. and Mann, M., *Nature* 405:837-846, 2000).

The present invention have endeavored to develop a novel binding protein to HCCR-1 protooncogene-derived protein product using the yeast two-hybrid method, and found a novel human protooncogene KG-19 which expresses a protein that specifically binds to HCCR-2 as well as HCCR-1 protooncogene, respectively. This protooncogene KG-19 can be advantageously used in diagnosis, prevention and treatment of various cancers, e.g., leukemia, lymphoma, colon, breast, kidney, stomach, lung, ovary and uterine cancers.

### Summary of the Invention

Accordingly, an object of the present invention is to provide a novel protoncogene and a biologically active fragment thereof.

Other objects of the present invention are to provide:
a recombinant vector containing said protooncogene or a biologically active fragment thereof and a microorganism transformed therewith;
a protein encoded in said protooncogene and a biologically active fragment thereof;
a kit for diagnosing cancer which comprising said protooncogene or a biologically active fragment thereof;
a kit for diagnosing cancer which comprising said protein or a biologically active fragment thereof;
an antisense gene having a base sequence complementary to that of said protooncogene or a fragment thereof;
a process for treating or preventing cancer by using said antisense gene; and
a process for screening anticancer drug candidate by using said protooncogene or a fragment thereof.

In accordance with one aspect of the present invention, there is provided a novel protooncogene having the nucleotide sequence of SEQ ID NO: 1 or a biologically active fragment thereof.

In accordance with another aspect of the present invention, there is provided a recombinant vector containing said protooncogene or a biologically active fragment thereof and a microorganism transformed with said vector.

In accordance with still another aspect of the present invention, there is provided a protein having the amino acid sequence of SEQ ID NO: 2 or a biologically active fragment thereof derived from said protooncogene or a fragment thereof.

### Brief Description of the Drawings

The above and other objects and features of the present invention will become apparent from the following description of the invention, when taken in conjunction with the accompanying drawings which respectively show;
Fig. 1a : the results of northern blot analyses for KG-19 protoongene expressed in normal human 12-lane multiple tissues (brain, hear t, skeletal muscle, colon, thymus, spleen, kidney, liver, small intestine, placenta, lung and peripheral blood leukocyte);
Fig. 1b: the results obtained after hybridizing the same sample of Fig. 1a hybridized with β-actin;
Fig. 2a : the results of northern blot analyses for KG-19 protoongene expressed in human cancer cell lines (promyelocytic leukemia HL-60 cell, HeLa cervical cancer cell, chronic myelogenous leukemia K-562 cell, lymphoblastic leukemia MOLT-4 cell, Burkitt's lymphoma Raji cell, SW480 colon cancer cell, A549 lung cancer cell and G361 melanoma cell);.
Fig. 2b : the results obtained after hybridizing the same sample of Fig. 2a with β-actin;
Fig. 3a : the results of northern blot analyses for KG-19 protoongene expressed in ovarian cancer tissues;
Fig. 3b : the results obtained after hybridizing the same sample of Fig. 3 a with β-actin;
Fig. 4a : the results of northern blot analyses for KG-19 protoongene expressed in breast cancer tissues;
Fig. 4b : the results obtained after hybridizing the same sample of Fig. 4a with β-actin;
Fig. 5a : the results of northern blot analyses for KG-19 protoongene expressed in lung cancer tissues;
Fig. 5b : the results obtained after hybridizing the same sample of Fig. 4a with β-actin;
Fig. 6a : the phase-contrast feature of monolayer-cultured wild type 293 human embryonic kidney cells;
Fig. 6b : the phase-contrast feature of monolayer-cultured KG-19-transfected 293 human embryonic kidney cells;
Fig. 7 : hematoxylin-eosin staining of monolayer-cultured KG-19-transfected 293 human embryonic kidney cells;
Fig. 8 : tumorigenicity of KG-19 cells in nude mouse;
Fig. 9 : sodium dodecyl sulfate (SDS)-PAGE results showing protein expression patterns before and after the IPTG induction in KG-19-transfected 293 human embryonic kidney cells;

### Detailed Description of the Invention

The novel protooncogene of the present invention, designated KG-19, consists of 772 base pairs and has the DNA sequence of SEQ ID NO: 1. This KG-19 protooncogene specifically binds to human cervical cancer proto-oncogene 1 (hereinafter "HCCR-1 protooncogene") described in Korea Patent Application No. 2000-16757 and HCCR-2 protooncogene described in Korea Patent Application No. 2000-71202.

In SEQ ID NO: 1, the open reading frame corresponding to base Nos. 138 to 638 is a full-length protein encoding region and the predicted amino acid sequence derived therefrom is shown in SEQ ID NO: 2 which consists of 166 amino acids ("KG-19 protein"). However, in consideration of the degeneracies of codons and the preferred codons in a specific animal wherein the protooncogene of the present invention is to be expressed, various changes and modifications of the DNA sequences of SEQ ID NO: 1 may be made, e.g., in the coding area thereof without adversely altering the amino acid sequence of the expressed protein, or in the non-coding area without adversely affecting the expression of the protooncogene. Therefore, the present invention also includes, in its scope, a polynucleotide having substantially the same base sequence as the inventive protooncogene, and a biologically active fragment thereof. As used herein, "substantially the same polynucleotide" refers to a polynuleotide whose base sequence shows 80% or more, preferably 90% or more, most preferably 95% or more homology to the protooncogene of the present invention.

The protein expressed from the protooncogene of the present invention consists of 166 amino acids and has the amino acid sequence of SEQ ID NO:2. The molecular weight of this protein is about 19 kDa. However, various substitution, addition and/or deletion of the amino acid residues of protein may be performed without adversely affecting the protein's function. Further, a portion of the protein may be used, when a specific purpose is to be fulfilled. These modified amino acids and fragments thereof are also included in the scope of the present invention. Therefore, the present invention includes, in its scope, a polypeptide having substantially the same amino acid sequence as the protein derived from the oncogene of the present invention and a biologically active fragment thereof. As used herein, "substantially the same polypeptide" refers to a polypeptide whose amino acid sequence shows 80% or more, preferably 90% or more, most preferably 95% or more homology to the amino acid sequence of SEQ ID NO: 2.

The protooncogene, or the protein, of the present invention can be obtained from human cancer tissues or synthesized using a conventional DNA or peptide synthesis method. Further, the gene thus prepared may be inserted to a conventional vector to obtain an expression vector, which may, in turn, be introduced into a suitable host, e.g., a microorganism such as an *E*. *coli* or yeast, or an animal cell such as a mouse or human cell. Transformed cells with KG-19 protooncogene or a fragment thereof is designated "KG-19 cells".

A transformed host may then be used in producing the inventive DNA or protein on a large scale. For example, *E. coli* XL1-Blue is transformed with expression vector pGEM-T (Promega, USA) containing the inventive KG-19 gene to obtain an *E. coli* transformant designated XL1-Blue MRA/pGEM-T/KG-19 which was deposited on September 18, 2000 with the Korean Collection for Type Cultures (KCTC) (Address: Korea Research Institute of Bioscience and Biotechnology (KRIBB), #52, Oun-dong, Yusong-ku, Taejon, 305-333, Republic of Korea) under the accession number KCTC 0826BP, in accordance with the terms of Budapest Treaty on the International Recognition of the Deposit of Microorganism for the Purpose of Patent Procedure.

In preparing a vector, expression-control sequences, e.g., promoter, terminator, self-replication sequence and secretion signal, are suitably selected and combined depending on the host cell used.

The overexpression of the protooncogene of the present invention occurs not in normal human tissues, e.g., brain, heart, skeletal muscle, colon, thymus, spleen, kidney, liver, small intestine, placenta, lung and peripheral blood leukocyte, but in cancer tissues, e.g., leukemia, lymphoma, breast, kidney, ovary, lung and stomach, skin cancer cell lines. This suggests that the protooncogene of the present invention induces the above-mentioned cancers. Further, when a normal 293 human embryonic kidney (HEK) cell is transfected with the protooncogene of the present invention, an abnormal cell is produced. Morphological characterizations with optical and electronic microscopes show that the abnormal cell has the form of a tumor cell. By using hematoxylin-eosin dye method, it can be confirmed that the tumor cell is cancerous (carcinoma).

When the normal 293 HEK cells transfected with the protooncogene of the present invention is injected into the back of nude mice, tumorigenesis is observed after about 14 days from the injection, the tumor size becoming 2.0 cm × 2.0 cm in 30 days.

Therefore, the protooncogene of the present invention is believed to be a factor common to all forms of various cancers, and it can be advantageously used in the diagnosis of various cancers and the production of a transgenic animal as well as in an antisense gene therapy and anticancer drug development.

A diagnostic method that can be performed using the protooncogene of the present invention may comprise, for example, the steps of hybridizing nucleic acids separated from the body fluid of a subject with a probe containing the protooncogene of the present invention or a fragment thereof, and determining whether the subject has the protooncogene by using a conventional detection method in the art. The presence of the protooncogene may be easily detected by labeling the probe with a radioisotope or an enzyme. Therefore, a cancer diagnostic kit containing the protooncogene of the present invention or a fragment thereof is also included in the scope of the present invention.

A transgenic animal produced by introducing the protooncogene of the present invention into a mammal, e.g., a rat, is also included in the scope of the present invention. In producing such a transgenic animal, it is preferred to introduce the inventive protooncogene to a fertilized egg of an animal before the 8th cell cycle stage. The transgenic animal can be advantageously used in screening for carcinogens or anticancer agents such as antioxidants.

The present invention also provides an antisense gene which is useful in a gene therapy. As used herein, the term "an antisense gene" means a polynucleotide comprising a base sequence which is fully or partially complementary to the sequence of the mRNA which is transcribed from the protooncogene having the base sequence of SEQ ID NO: 1 or a fragment thereof, said nucleotide being capable of preventing the expression of the open reading frame (ORF) of the protooncogene by way of attaching itself to the protein-binding site of mRNA.

The present invention also includes within its scope a process for treating or preventing cancer in a subject by way of administering a therapeutically effective amount of the inventive antisense gene thereto.

In the inventive antisense gene therapy, the antisense gene of the present invention is administered to a subject in a conventional manner to prevent the expression of the protooncogene. For example, the antisense oligodeoxynucleotide (ODN) is mixed with a hydrophobized poly-L-lysine derivative by electrostatic interaction in accordance with the method disclosed by Kim, J. S. et al. (*J. Controlled Release* **53**: 175-182, 1998) and the resulting mixed antisense ODN is administered intravenously to a subject.

The present invention also includes within its scope an anti-cancer composition comprising the antisense gene of the present invention as an active ingredient, in association with pharmaceutically acceptable carriers, excipients or other additives, if necessary. The pharmaceutical composition of the present invention is preferably formulated for administration by injection.

The amount of the antisense gene actually administered should be determined in light of various relevant factors including the condition to be treated, the chosen route of administration, the age and weight of the individual patient, and the severity of the patient's symptoms.

The protein expressed from the inventive protooncogene may be used in producing an antibody useful as a diagnostic tool. The antibody of the present invention may be prepared in the form of a monoclonal or polyclonal antibody in accordance with any of the methods well known in the art by using a protein having the amino acid sequence of SEQ ID NO: 2 or a fragment thereof. Cancer diagnosis may be carried out using any of the methods known in the art, e.g., enzyme linked immunosorbent assay (ELISA), radioimmunoassay (RIA), sandwich assay, immunohistochemical staining, western blot or immunoassay blot on polyacrylic gel, to asses whether the protein is expressed in the body fluid of the subject. Therefore, a cancer diagnostic kit containing the protein having the amino acid sequence of SEQ ID NO: 2 or a fragment thereof is also included in the scope of the present invention.

A continuously viable cancer cell line may be established by using the protooncogene of the present invention, and such a cell line may be obtained, for example, from tumor tissues formed on the back of a nude mouse by injecting fibroblast cells transformed with the protooncogene of the present invention. The cell lines thus prepared may be advantageously used in searching for anti-cancer agents.

The present invention also includes within its scope a process for screening anticancer drug candidate by using said protooncogene or a fragment thereof.

After characterizing the role of the inventive protooncogene and its protein product in the tumorigenesis, they can be used for inhibiting the oncogene or oncoprotein by administrating antisense oligodeoxynucleotide targeting the genes or antibodies targeting the proteins. Using the characterized role of the inventive protooncogene, it can be also found the interacting molecules that bind to known oncoproteins, and then, designed inhibitors, anticancer drug candidate, which can break down the interacting molecules.

The following Examples and Test Examples are given for the purpose of illustration only, and are not intended to limit the scope of the invention.

### Example 1: Yeast two hybrid assay

To find a binding protein to human protooncogene HCCR-1 (Genebank Accession No: AF195651) protein product, yeast two hybrid assay was performed by using MATCHMAKER LexA Two-Hybrid System (Clontech Laboratories, Inc., USA) according to the previously reported procedure (Golemis, E. A., et al., *Current Protocols in Molecular Biology,* John Wiley & Sons, Inc. Chapters 20.0 and 20.1, 1996).

Cell lines and vectors used in the following experiment are listed in Catalog No. K1609-1 Kit of Clontech Laboratories, Inc.

Yeast strain EGY48 was transformed with vector p8op-lacZ and cultured in an SD/-uracil/glucose plate, a synthetic dropout medium having no uracil. The colony that grew in this plate was selected, cultured in an SD/-uracil/glucose medium, and transformed with a vector prepared by inserting HCCR-1 gene into the *Bam*HI and *Sal*I sites of vector pLexA as a 'bait plasmid'.

To examine the expression of HCCR-1 gene cloned in vector pLexA, western blotting using LexA antibody was performed. The result showed a 64 ~ 65 kDa band, respectively.

The colony expressing HCCR-1 gene was cultured in an SD/-uracil, - histidine/glucose medium and transformed with vector pBD42AD which is human fetal brain-derived AD (activation domain) fusion library. To confirm the binding of the bait with the library, colony lifting assay (Breeden, L. and Nasmyth, K., *Cold Spring Harbor Symposium Quant. Bio.* 50:643-650, 1985) was performed. If the bait interacts with the library, a blue colony would form in an X-gal plate. DNA was purified from the yeast cultured using glass beads, and *E. coli* KC8 was transformed with the purified DNA by electrophoration and spreading on an M9 minimal medium to select transformants.

The nucleotide sequence of full-length KG-19 cDNA clone was registered at GenBanlc under Accession No. AF283671.

In SEQ ID NO: 1, the full open reading frame of KG-19 corresponding to base Nos. 136 to 638 is a protein encoding region and the predicted amino acid sequence derived therefrom is shown in SEQ ID NO: 2 which consists of 166 amino acids.

The full-length KG-19 cDNA was inserted in the multicloning site of vector pGEM-T-easy (Promega, USA) using DNA ligase to obtain a recombinant vector expressing KG-19 gene. *E. coli* XL1-Blue MRA (Stratagene, USA) was transformed with the recombinant vector to obtain a transformed *E. coli* designated XL1-Blue MRA/pGEM-T-easy KG-19, which was deposited with Korean Collection for Type Cultures (Address: #52, Oun-dong, Yusong-ku, Taejon 305-333, Republic of Korea) on September 18, 2000 under the accession number of KCTC 0862BP.

### Example 3: Northern blot analysis

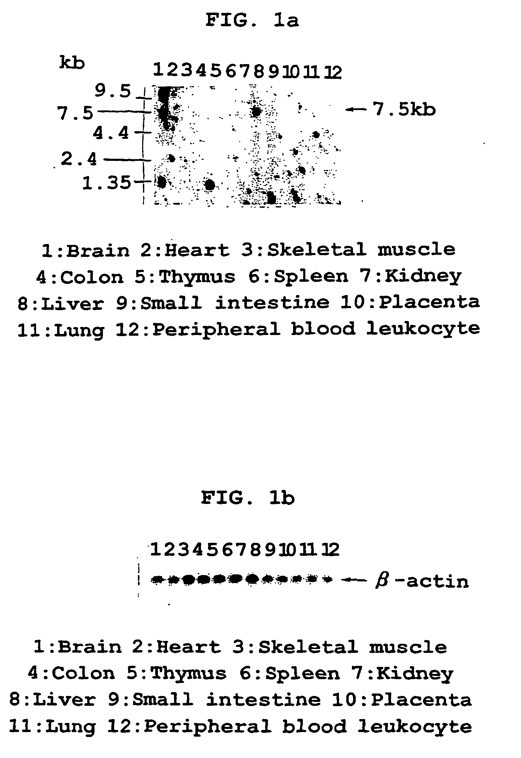

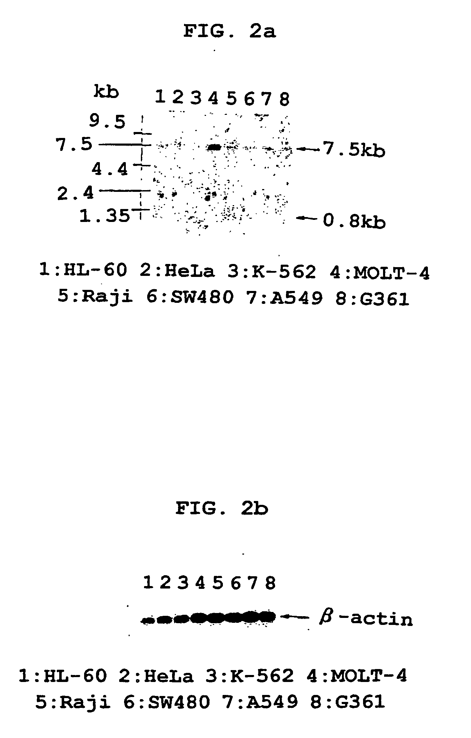

To determine the expression level of KG-19 gene in various normal tissues and cancer tissues, northern blot analyses were also carried out as recommended by the supplier (Clontech) using a commercial sample containing purified DNA's of normal human 12-lane multiple-tissues blotted on a nylon membrane (Figs. 1a and 1b) and also using a human cancer cell line sample containing purified RNAs of 8 kinds of cancer cells blotted on a nylon membrane (Figs. 2a and 2b).

The blots were hybridized overnight at 68°C with ³²P-labeled random-primed KG-19 cDNA probe which was prepared using a Rediprime II random prime labeling system (Amersham, England). The northern blot analysis was repeated twice, and the results were quantified by densitometry. The same blots were hybridized with a β-actin probe to confirm mRNA integrity.

Fig. 1a shows the northern blot analysis result of normal human 12-lane multiple tissues, i.e., brain, heart, skeletal muscle, colon, thymus, spleen, kidney, liver, small intestine, placenta, lung, and peripheral blood leukocyte, using KG-19 cDNA probe; and Fig. 1b, the same blot hybridized with a β-actin probe. As can be seen in Fig. 1a, the 0.8 kb transcript of the two KG-19 mRNA transcripts is not detected in any of the normal tissues, while the 7.5 kb transcript, which is regarded as KG-19 protooncogene-containing transcript before splicing, is weakly expressed in only normal kidney tissue.

Fig. 2a shows the northern blot analysis for KG-19 gene expressed in human cancer cell lines, i.e., promyelocytic leukemia HL-60 cell, HeLa cervical cancer cell, chronic myelogenous leukemia K-562 cell, lymphoblastic leukemia MOLT-4 cell, Burkitt's lymphoma Raji cell, SW480 colon cancer cell, A549 lung cancer cell, and G361 melanoma cell, using KG-19 cDNA probe; and Fig. 2b, the same blot hybridized with a β-actin probe. As can be seen in Fig. 2a, the 0.8 kb transcript of KG-19 is detected at the Burkitt's lymphoma Raji and promyelocytic leukaemia HL-60, and the 7.5 kb transcript is also expressed at a high level in the promyelocytic leukaemia HL-60, HeLa cervical cancer cell, chronic myelogenous leukaemia K-562, lymphoblastic leukaemia MOLT-4, Burkitt's lymphoma Raji, SW480 colon cancer cell, A549 lung cancer cell, and G361 melanoma cell. MOLT-4 and Raji, in particular, show higher transcription levels as compared with other cancer cells.

Further, northern blotting analyses of the ovary, breast and lung cancer tissues and their normal counterparts were carried out to confirm the expression of KG-19 gene. Total RNAs were purified from ovary, breast, and lung tissues of healthy normal people, and from ovarian cancer, breast cancer, and lung cancer tissues of cancer patient, using RNeasy total RNA Kit (Qiagen Inc., Germany). 20 µg each of the total RNAs were denatured and then electrophoresed through 1% formaldehyde agarose gel and transferred to nylon membranes (Boehringer-Mannheim, Germany). The blots were hybridized at 68°C with ³²P-labeled random-primed KG-19 total cDNA probe which was prepared using a Rediprime II random prime labeling system (Amersham, England). The northern blot analysis was consistently repeated twice and the result was quantified by densitometry. The same blots were hybridized with a β-actin probe to confirm mRNA integrity.

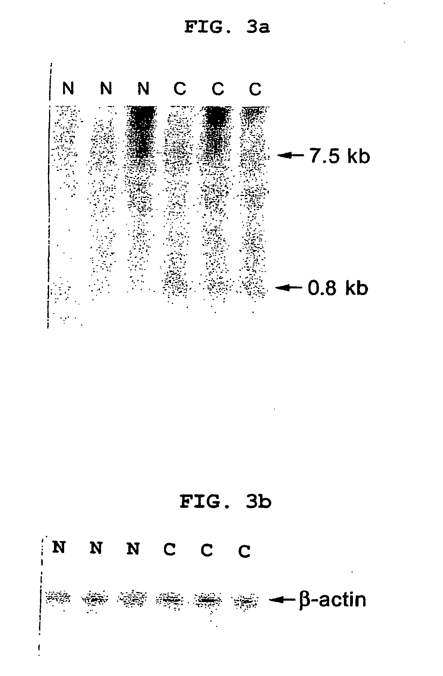

Fig. 3a shows the northern blot analysis result of normal ovary tissue and ovarian cancer tissue using KG-19 cDNA probe; and Fig. 3b, the same blot hybridized with a β-actin probe. As shown in Fig. 3a, the 0.8 kb transcript of KG-19 is expressed at a high level in the ovarian cancer tissues, while the expression of KG-19 gene is barely observable in the normal ovary tissues. In Figs. 3a and 3b, N lane and C lane represent normal ovary tissue and ovarian cancer tissue, respectively.

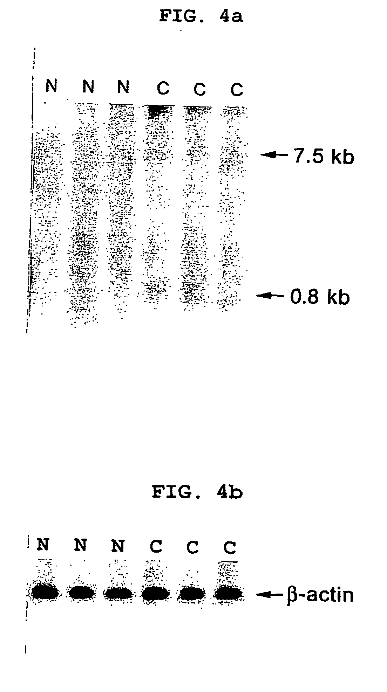

Fig. 4a shows the northern blot analysis result of normal breast tissue and breast cancer tissue using KG-19 cDNA probe; and Fig. 4b, the same blot hybridized with a β-actin probe. As shown in Fig. 4a, the 0.8 kb transcript of KG-19 is expressed at a high level in the ovarian cancer tissue, while the expression of KG-19 gene is barely observable in the normal breast tissue. In Figs. 4a and 4b, N lane and C lane represent normal breast tissue and breast cancer tissue, respectively.

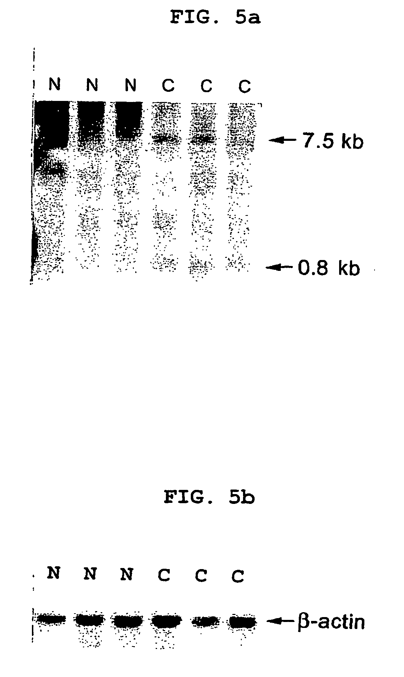

Fig. 5a shows the northern blot analysis result of normal lung tissue and lung cancer tissue using KG-19 cDNA probe; and Fig. 5b, the same blot hybridized with a β-actin probe. As shown in Fig. 5a, the 0.8 kb and the 7.5 kb transcripts of KG-19 are expressed at high levels in the lung cancer tissue, while the expression of KG-19 gene is barely observable in the normal lung tissue. In Figs. 5a and 5b, N lane and C lane represent normal lung tissue and lung cancer tissue, respectively.

### Example 4: Construction of expression vectors and transformation of animal cells

### (Step 1) Preparation of a vector containing KG-19

An expression vector containing the coding region of KG-19 was constructed as follows.

First, fusion vector pCDNA3 (Invitrogen, USA)-glutathione-S transferase (GST) was constructed by inserting GST gene into pcDNA3 eucaryotic expression vector by *Hin*dIII/*Kpn*I digestion. KG-19 gene having the total coding sequence (corresponding to base Nos. 121-674 in SEQ ID NO: 1) was digested with *Bam*HI/*Xba*I*,* and, then, the *Bam*HI/*Xba*I fragment containing the full length KG-19 coding sequence was inserted into the *Bam*HI/*Xba*I-digested pCDNA3. Lipofectamine (Gibco BRL) was used to introduce the resulting vector pCDNA3/GST/KG-19 into 293 human embryonic kidney epithelial cells (ACTC CRL 1753, USA), followed by selection in a medium supplemented with G418 (Gibco, USA). The resulting 293 cells transfected with KG-19 was designated "KG-19 cells".

### (Step 2) 293 human embryonic kidney epithelial cells transfected with the KG-19 protooncogene

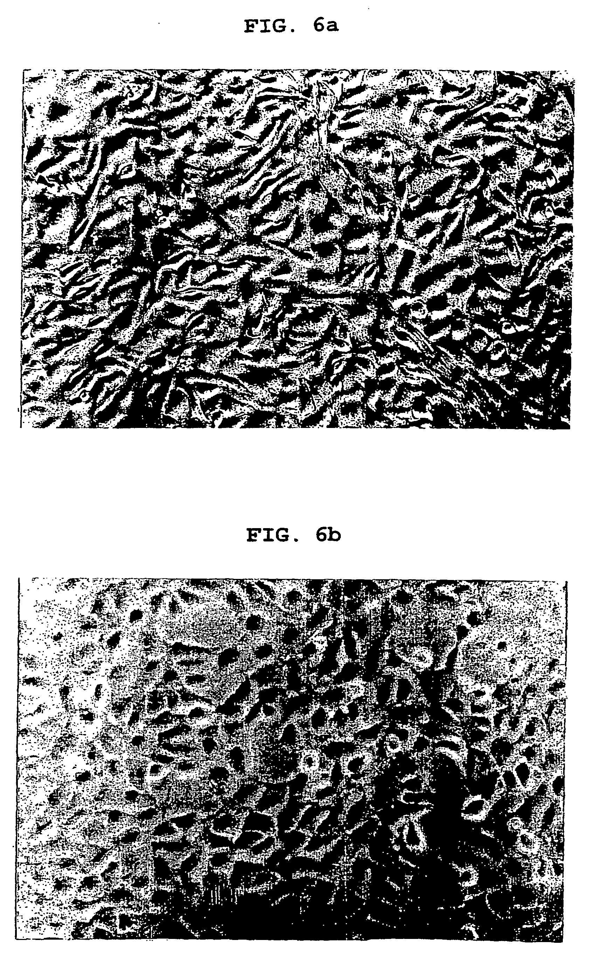

The wild type normal 293 cells and the 293 KG-19 cells obtained in Step 1 were cultured in a monolayer in DMEM media containing 10% serum, and their growth features were observed with a Phase-contrast microscopy (Olympus, USA). Figs. 6a and 6b show phase-contrast features of monolayer-cultured wild type 293 human embryonic kidney cells and KG-19-transfected 293 human embryonic kidney cells. As shown in Fig. 6a, the wild-type 293 cells proliferated as epithelial cells each has a spindle-shape having a long, fine nucleus and meager cytoplasm. In case of KG-19 cells, the cell shape changes into a polygonal form with an ovoid nucleus and plump cytoplasm, as shown in Fig. 6b.

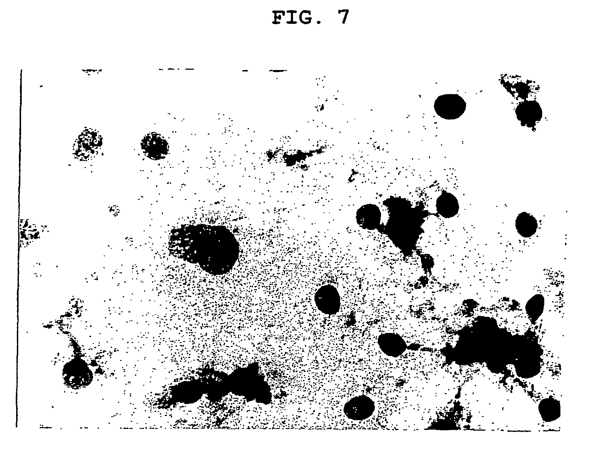

Monolayer cultured KG-19 cells, which is stained with hematoxylin-eosin (H & E), exhibit nuclear pleiomorphism, distinct nucleoli, granular chromatin patterns, tumor giant cells and atypical mitotic figures as shown in Fig. 7.

### Example 5: Tumorigenicity of KG-19 protooncogene in animal

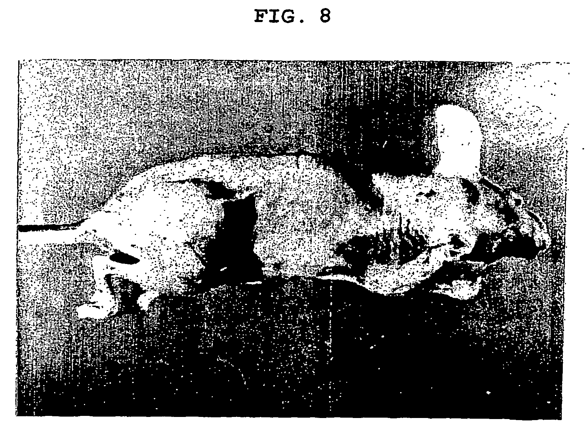

To analyze tumourigenicity, 5 × 10⁶ KG-19 cells were injected subcutaneously into the back of the trunk of 9 nude mice (5-week-old athymic nu/nu on BALB/c background). All 9 mice injected with KG-19 cells showed palpable tumors after 14days, the tumor size becoming 2.0 cm × 2.0 cm in 30 days as shown in Fig 8.

### Example 6: Determination of size of protein expressed after the transfection of E. coli with KG-19 protooncogene

The full-length coding sequence region of KG-19 protooncogene corresponding to base Nos. 138 to 638 of SEQ ID NO: 1 and encoding amino acid Nos. 1 to 166 of SEQ ID NO: 2, was inserted into *Bam*HI/*Not*I recognition site of the multiple cloning site of vector pGEX 4T-3 (Amersham Pharmacia Biotech., USA) fused with GST gene, and the resulting vector pGEX 4T-3/KG-19 was transfected into *E. coli* BL21 (ATCC 47092). The transfected *E*. *coli* was incubated using an LB broth medium in a rotary shaking incubator at 37°C for 16 hours, diluted by 1/100, and incubated for 3 hours. 1 mM isopropyl β-D-thiogalacto-pyranoside (IPTG, Sigma) was added thereto to induce the protein synthesis.

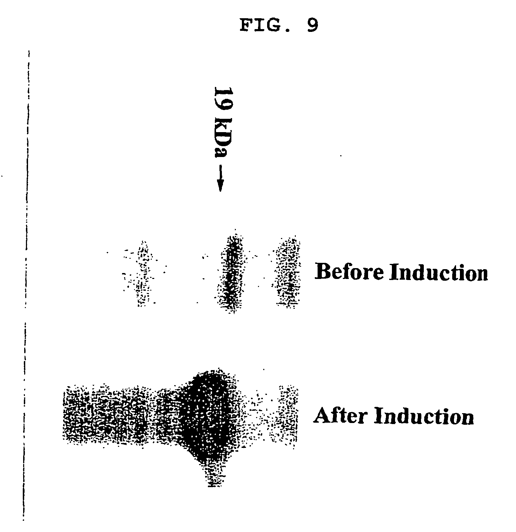

The *E*. *coli* cells in the culture were disrupted by sonication and subjected to gel electrophoresis using 12% sodium dodecyl sulfate (SDS) before and after the IPTG induction. Fig. 9 shows the SDS-PAGE results which exhibit a protein expression pattern of the *E. coli* BL21 strain transfected with vector pGEX 4T-3/KG-19. After the IPTG induction, a significant protein band was observed at about 45kDa. This 45kDa fused protein contained an about 261cDa GST protein which was expressed from the gene of vector pGEX 4T-3.

While the embodiments of the subject invention have been described and illustrated, it is obvious that various changes and modifications can be made therein without departing from the spirit of the present invention which should be limited only by the scope of the appended claims.

## Claims

1. A human KG-19 protooncogene having the base sequence of SEQ ID NO: 1 or a biologically active fragment thereof, wherein the latter has the protooncogenic activity of the full-length.

2. The protooncogene or a biologically active fragment of claim, 1, which is a fragment having a base sequence corresponding to base Nos. 138 to 638 of SEQ ID NO: 1.

3. A protein having the amino acid sequence SEQ ID NO: 2 or a biologically active fragment thereof.

4. A vector comprising the protooncogene or a biologically active fragment of claim 1.

5. A microorganism transformed with the vector of claim 4.

6. The microorganism of claim 5, which is *E. coli* XL1-Blue MRA/pGEM-T/KG-19 (Accession No.: KCTC 0862BP).

7. A process for preparing the protein or a biologically active fragment of claim 3 comprising culturing the microorganism of claim 5 or 6.

8. A kit for diagnosing cancer which comprises the protooncogene or fragment of claim 1 or 2.

9. A kit for diagnosing cancer which comprises the protein or biologically active fragment of claim 3.

10. An antisense gene having a base sequence which is complementary to the sequence of the full or partial mRNA transcribed from the protooncogene or biologically active fragment of claim 1 or 2 and being capable of binding the mRNA to inhibit the expression of said protooncogene or fragment.

11. A composition for preventing or treating cancer which comprises a therapeutically effective amount of the antisense gene of claim 10 and a pharmaceutically acceptable carrier.

## Patentansprüche

1. Humanes KG-19-Protoonkogen mit der Basensequenz von SEQ ID Nr.: 1 oder ein biologisch aktives Fragment davon, wobei letzteres die Protoonkogen-Aktivität der Vollängenform aufweist.

2. Protoonkogen oder ein biologisch aktives Fragment von Anspruch 1, welches ein Fragment mit einer Basensequenz, entsprechend Base Nr. 138 bis 638 von SEQ ID Nr.: 1, ist

3. Protein mit der Aminosäuresequenz SEQ ID Nr.: 2 oder ein biologisch aktives Fragment davon.

4. Vektor, umfassend das Protoonkogen oder ein biologisch aktives Fragment von Anspruch 1.

5. Mikroorganismus, transformiert mit dem Vektor von Anspruch 4.

6. Mikroorganismus von Anspruch 5, bei dem es sich um *E. coli* XL1-Blue MRA/pGEM-T/KG-19 (Zugangs-Nr.: KCTC 0862BP) handelt.

7. Verfahren zur Herstellung des Proteins oder eines biologisch aktiven Fragmentes von Anspruch 3, umfassend das Kultivieren des Mikroorganismus von Anspruch 5 oder 6.

8. Kit zum Diagnostizieren von Krebs, welches das Protoonkogen oder Fragment von Anspruch 1 oder 2 umfasst.

9. Kit zum Diagnostizieren von Krebs, welches das Protein oder biologisch aktive Fragment von Anspruch 3 umfasst.

10. Antisense-Gen mit einer Basensequenz, welche komplementär zu der Sequenz der vollständigen oder teilweisen mRNA ist, die von dem Protoonkogen oder biologisch aktiven Fragment von Anspruch 1 oder 2 transkribiert wird, und zur Bindung der mRNA zur Inhibierung der Expression des Protoonkogens oder Fragmentes in der Lage ist.

11. Zusammensetzung zur Verhinderung oder Behandlung von Krebs, welche eine therapeutisch wirksame Menge des Antisense-Gens von Anspruch 10 und einen pharmazeutisch annehmbaren Träger umfasst.

## Revendications

1. Protooncogène humain KG-19 ayant la séquence de bases de SEQ ID n°: 1 ou fragment biologiquement actif de celle-ci, ce dernier ayant l'activité protooncogénique de la séquence entière.

2. Protooncogène ou fragment biologiquement actif selon la revendication 1, qui est un fragment ayant une séquence de bases correspondant aux bases n°138 à 638 de SEQ ID n°: 1.

3. Protéine ayant la séquence d'aminoacides SEQ ID n°: 2 ou fragment biologiquement actif de celle-ci.

4. Vecteur comprenant le protooncogène ou un fragment biologiquement actif de la revendication 1.

5. Microorganisme transformé par le vecteur de la revendication 4.

6. Microorganisme de la revendication 5, qui est *E. coli* XL1-Blue MRA/pGEM-T/KG-19 (numéro d'ordre : KCTC 0862BP).

7. Procédé pour la préparation de la protéine ou d'un fragment biologiquement actif de la revendication 3, comprenant la culture du microorganisme de la revendication 5 ou 6.

8. Kit pour le diagnostic d'un cancer qui comprend le protooncogène ou le fragment de la revendication 1 ou 2.

9. Kit pour le diagnostic d'un cancer qui comprend la protéine ou le fragment biologiquement actif de la revendication 3.

10. Gène antisens ayant une séquence de bases complémentaire de la séquence de l'ARNm total ou partiel transcrit à partir du protooncogène ou du fragment biologiquement actif de la revendication 1 ou 2 et capable de se lier à l'ARNm pour inhiber l'expression dudit protooncogène ou dudit fragment.

11. Composition pour la prévention ou le traitement du cancer qui comprend une quantité thérapeutiquement efficace du gène antisens de la revendication 10 et un support pharmaceutiquement acceptable.


### SEQUENCE LISTINGS

<110> KIM, Jin Woo
<120> Human protooncogene and protein encoded therein
<130> PCA/11049/KJW/PCT
<160> 2
<170> KOPATIN 1.5
<210> 1
   <211> 772
   <212> DNA
   <213> Homo sapiens
<400> 1
   aggcggaaga acagctaccc gccgcacggt caacaccttc tcccagagcg tcagcaagcc 60
   tgttcggcga ggacaacgtg cgcgccgctc agaagttctt ggccaggctg actgagagat 120
   ttgtgctggg agtggatatg ttcgtggaga cactgtggaa agtctggacc gagctcttgg 180
   atgttcttgg acttgacgtc tccaacctgt cccagtattt cagcccagcc tcggtgtcca 240
   gcagcccggc ccgcgcgctc ctgctggtcg gcgtcgtcct cctggcctac tggttcttgt 300
   ccctgaccct gggcttcact ttcagcgtcc tgcacgtggt gttcggccgc ttcttctgga 360
   tcgtgcgggt cgtcctgttt tccatgtcct gcgtgtacat cctgcacaag tacgagggcg 420
   agccggagaa cgcggtgctg ccgctgtgct tcgtggtggc cgtctacttc atgaccgggc 480
   ccatgggctt ctactggcga agcagtccca gcggccccag caaccccagc aaccccagcg 540
   tggaggagaa gctggagcac ctggagaagc aggtcagact gctcaacatc cgtctcaacc 600
   gggtgctcga gagcctggac cgctccaagg acaagtgaag gtcagccggc cgggcgggtc 660
   cacagttacc agcacgctgt ctcagaaaac gaaaacggag gaaaaaaacc ccaaacccca 720
   aacaatctta ataaacacga ctgagcaaaa aaaaaaaaaa aaaaaaaaaa aa 772
<210> 2
   <211> 166
   <212> PRT
   <213> Homo sapiens
<400> 2
   Met Phe Val Glu Thr Leu Trp Lys Val Trp Thr Glu Leu Leu Asp Val
   1 5 10 15
   Leu Gly Leu Asp Val Ser Asn Leu Ser Gln Tyr Phe Ser Pro Ala Ser
   20 25 30
   Val Ser Ser Ser Pro Ala Arg Ala Leu Leu Leu Val Gly Val Val Leu
   35 40 45
   Leu Ala Tyr Trp Phe Leu Ser Leu Thr Leu Gly Phe Thr Phe Ser Val
   50 55 60
   Leu His Val Val Phe Gly Arg Phe Phe Trp Ile Val Arg Val Val Leu
   65 70 75 80
   Phe Ser Met Ser Cys Val Tyr Ile Leu His Lys Tyr Glu Gly Glu Pro
   85 90 95
   Glu Asn Ala Val Leu Pro Leu Cys Phe Val Val Ala Val Tyr Phe Met
   100 105 110
   Thr Gly Pro Met Gly Phe Tyr Trp Arg Ser Ser Pro Ser Gly Pro Ser
   115 120 125
   Asn Pro Ser Asn Pro Ser Val Glu Glu Lys Leu Glu His Leu Glu Lys
   130 135 140
   Gln Val Arg Leu Leu Asn Ile Arg Leu Asn Arg Val Leu Glu Ser Leu
   145 150 155 160
   Asp Arg Ser Lys Asp Lys
   165